# EUROPEAN PATENT SPECIFICATION

(11) **EP 4 341 617 B1**
(45) Date of publication and mention of the grant of the patent: **17.06.2026**
(21) Application number: 22733728.4
(22) Date of filing: 23.05.2022
(51) Int. Cl.: F24F 8/24, F24F 8/28, C11D 3/00, C12N 1/00, A61L 2/22, A61L 9/14

(54) **DISPENSING APPARATUS, USE AND METHOD OF SANITISATION THEREOF**
DISPENSIERGERÄT, VERWENDUNG UND VERFAHREN ZUR DESINFEKTION
APPAREIL DE DISTRIBUTION, UTILISATION ET PROCEDE DE SANITISATION DE CELUI-CI

(30) Priority: 21.05.2021 IT 202100013319
(43) Date of publication of application: 27.03.2024
(73) Proprietor: Proair Global Company Limited, Hong Kong 999077 (HK)
(72) Inventor: LITTINI, Giuseppe, 6900 Lugano (CH)
(74) Representative: Metida
(86) International application number: PCT/IB2022/054809
(87) International publication number: WO 2022/243986

(56) References cited:
- WO-A1-03/008001
- CN-A- 110 882 621
- DE-A1- 19 821 504
- US-A1- 2015 202 388

## Description

The present invention relates to a dispensing apparatus comprising (i) a liquid solution of bacteria, preferably live and viable bacteria, even more preferably live and viable bacteria having an intact cell membrane, and (ii) a dispensing device. Further, the present invention relates to a use of said dispensing apparatus for sanitising surfaces and/or environments, preferably indoor environments. Furthermore, the present invention relates to an air conditioning system comprising said dispensing apparatus.

Finally, the present invention relates to a method of sanitisation implemented by said dispensing apparatus, or by said air conditioning system.

Document US 2015/0202388 A1 discloses a system for dispersing a solution containing probiotic bacteria by atomising the solution with pressurised air in a venturi configuration and delivering the atomised solution into an HVAC system or a room. In this known system, the solution is drawn from a solution chamber and atomised directly by the venturi effect, without the use of a peristaltic pump feeding the solution to a separate mixing chamber provided with a dispensing nozzle for the resulting liquid-gas mixture in the form of an atomised jet.

An air conditioner is a thermal machine designed to lower or raise the temperature in an environment (e.g in a room or in the passenger compartment of a vehicle) and is therefore one of the possible benefits to combat heat and mugginess or, conversely, cold and humidity.

The operating principle of air conditioners is the so-called "heat pump": by transferring heat to, or absorbing heat from, a heat exchange fluid contained in a circuit, energy is removed from one side of the circuit to be transferred to another side of the same circuit.

The construction elements of an air conditioning system are: compressor, evaporator, condenser, lamination valve, reversible valve, and air filter.

The compressor creates a pressure difference that allows the circuit (and thus the thermodynamic cycle) to function, as it sucks in and compresses the heat exchange fluid from the evaporator, and pushes this fluid into the condenser. In the condenser, this fluid condenses under pressure, releasing the absorbed heat. After the condenser, the fluid passes through the lamination valve, which is a throttling organ delegated to degrade the pressure energy in friction, and which produces an irreversible expansion of the condensed heat exchange fluid. Fluidically downstream of the lamination valve, the heat exchange fluid flows into the evaporator to restart its cycle. Thus, the heat exchange fluid changes its physical state in the two heat exchangers, i.e. in the evaporator (from a liquid state to a gas state) and in the condenser (from a gas state to a liquid state).

The reversible valve is the construction element delegated to reverse the flow direction of the heat exchange fluid in the circuit, thus allowing heat to be taken from the environment (lowering its temperature), or heat to be introduced into the same environment (raising its temperature).

Finally, the air filter is necessary to clean the air - thermally treated in the air conditioner - from solid particles and suspended microorganisms.

One of the critical issues in air conditioned is system maintenance, and more specifically the cleaning of air filters. These filters are an ideal environment for a colonisation by pathogenic microorganisms, such as bacteria (e.g. of the *Legionella* genus) and moulds (e.g. of the *Penicillium* genus), and provide a warm, moist environment that supports their proliferation. Serious human health problems can result from contaminated air filters, as pathogenic micro-organisms - under the forced drive of the air - can pass through ventilation ducts, be released into the environment, settle on surfaces, and be breathed in or come into contact with the eyes.

Especially in workplaces, contamination of air conditioning systems frequently causes cases of "sick building syndrome" (or SBS), a pathological condition that occurs when from 20% to 30% of workers suffer symptoms - such as migraine, drowsiness, nasal obstruction, itching, irritation, conjunctivitis, eye burning and respiratory disorders - but without specific causes or diseases being identified.

The Applicant, after lengthy and intensive research and development activity, has developed a dispensing apparatus, an air conditioning system and a sanitising method capable of providing an adequate response to the existing limitations, inconveniences and problems.

In particular, the present apparatus, system and method are designed to atomise a liquid solution containing bacteria, preferably live and viable bacteria, even more preferably live and viable bacteria having an intact cell membrane, wherein such bacteria remain viable during atomisation of the liquid solution, so as to be able to survive, replicate and colonise the environment and surfaces.

The present invention thus makes it possible to re-establish or achieve a biological balance in the air, in environments, preferably indoor environments and/or on surfaces, thereby drastically reducing the concentration of harmful or pathogenic bacteria and allergens, and helping to create a permanently healthy ecosystem.

Accordingly, it is an object of the present invention a dispensing apparatus comprising a liquid solution of preferably live and viable bacteria, even more preferably live and viable bacteria having intact cell membranes, and a dispensing device, having the characteristics as defined in the accompanying claims.

It is also an object of the present invention a use of said dispensing apparatus for sanitising surfaces and/or environments, having the characteristics as defined in the accompanying claims.

It is also an object of the present invention an air conditioning system comprising said dispensing apparatus, having the characteristics as defined in the accompanying claims.

It is a further object of the present invention a method of sanitising implemented by said dispensing apparatus, or by said air conditioning system, having the characteristics as defined in the accompanying claims.

Preferred embodiments of the present invention will be described herein by way of example, and therefore not limitative, with the aid of the attached drawings, wherein:
- Figures 1 and 2 show, respectively, a side and front view of a part of a dispensing device, which is the object of the present invention, according to a possible embodiment;

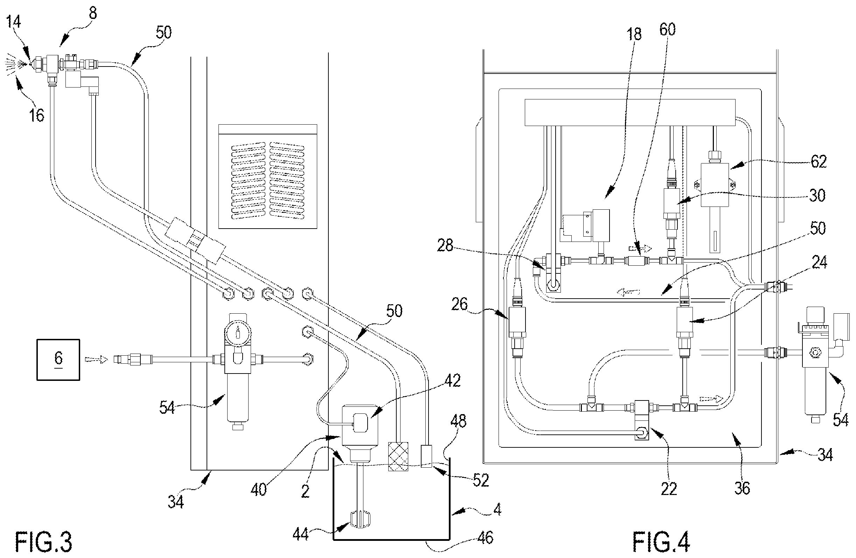

- Figures 3 and 4 illustrate a detail of the lower part (containing the hydraulic parts) of the dispensing device according to Figures 1 and 2, in another embodiment, in side and frontal section view, respectively;
- Figure 5 represents a functional diagram of the hydraulic part of the dispensing device, according to a possible embodiment;
- Figure 6 illustrates a possible shape of an atomised jet exiting from dipensing nozzle, according to a possible embodiment of said nozzle, wherein sections A, B, C indicate diameters of said jet at distances from the nozzle of 15 cm, 38 cm and at the end of the jet (when the latter assumes a turbulent shape), respectively.

Therefore, it is an object of the present invention a dispensing apparatus, marked - in its entirety - with reference number 1 in the attached drawings.

The dispensing apparatus comprises: (a) a liquid solution of probiotic bacteria 2 having intact cell membranes (and therefore viable); and (b) a dispensing device 10.

In the present description, the term "viable" means that a percentage from 90% to 100% of the total probiotic bacteria in the liquid solution, preferably from 95% to 99%, even more preferably from 96% to 98.5%, has an intact cell membrane, and is therefore "living" regardless of its ability to replicate.

Said probiotic bacteria having intact cell membranes are preferably non-spore-forming bacteria.

Preferably, said probiotic bacteria having intact cell membranes are selected from bacteria of the genus *Lactobacillus, Bifidobacterium, Streptococcus, Lactococcus, Akkermansia, Intestinimonas, Eubacterium, Faecalibacterium, Neisseria, Roseburia, Cutibacterium,* and mixtures thereof; more preferably from bacteria of the genus *Lactobacillus, Bifidobacterium,* and mixtures thereof

Even more preferably, said probiotic bacteria having intact cell membranes are selected from the group comprising or, alternatively, consisting of: *Lactobacillus acidophilus, Lactobacillus buchneri, Lactobacillus fermentum, Lactobacillus salivarius subsp. salivarius, Lactobacillus crispatus, Lactobacillus paracasei subsp. paracasei, Lactobacillus gasseri, Lactobacillus plantarum, Lactobacillus delbrueckii subsp. bulgaricus, Lactobacillus delbrueckii subsp. delbrueckii, Lactobacillus rhamnosus, Lactobacillus pentosus, Lactobacillus fermentum, Lactobacillus brevis, Lactobacillus plantarum, Lactobacillus casei, Lactobacillus reuteri, Lactobacillus johnsonii, Bifidobacterium adolescentis, Bifidobacterium animalis subsp. lactis, Bifidobacterium breve, Bifobacterium catenulatum, Bifobacterium pseudocatenulatum, Bifidobacterium bifidum, Bifidobacterium lactis, Bifidobacterium infantis, Bifidobacterium longum, Akkermansia munichipila, Intestinimonas butyriciproducens, Eubacterium hallii, Faecalobacterium prausnitzii, Neisseria lactamica, Roseburia hominis, Cutibacterium acnes,* and mixtures thereof.

Said probiotic bacteria having intact cell membranes are preferably freeze-dried bacteria reconstituted in the liquid solution. Said liquid solution is preferably an aqueous liquid solution of probiotic bacteria 2, more preferably an aqueous liquid solution of sodium chloride.

The aqueous liquid solution of sodium chloride preferably includes, in addition to the probiotic bacteria having intact cell membranes, sodium chloride in an amount from 0.1% to 15% by weight, preferably from 0.2% to 10% by weight, with respect to the total weight of said aqueous liquid solution.

The dispensing device 10 comprises: (i) a reservoir 4 accommodating said liquid solution of probiotic bacteria 2; (ii) a pressurised gas source 6; (iii) a dispensing body (or dispensing gun) 8; and (iv) at least one peristaltic pump 18.

The dispensing device 10 is preferably only partially enclosed in a device casing 34, preferably made of a metal material, more preferably steel, even more preferably stainless steel (e.g. AISI 304).

In this description, the expression "only partially enclosed" means that not all parts of the dispensing device 10 are contained in the device casing 34. By way of example, at least the reservoir 4 and the dispensing body 8 are preferably outside the device casing 34.

The device casing 34 preferably delimits at least two separate casing compartments 36, 38, a first casing compartment 36 (or lower compartment) accommodating the hydraulic parts of the device, and a second casing compartment 38 (or upper compartment) accommodating the electrical parts of the device. The separate casing compartments 36, 38 are preferably closed by at least one opening casing door 64.

Preferably, the dispensing device 10 comprises first temperature detection means 62 for detecting the temperature in the first casing compartment 36 and/or the second casing compartment 38. The first temperature detection means 62 therefore serve to signal any, abnormal changes in temperature in one or both of said compartments 36, 38.

The reservoir 4 has a volume commensurate with the volume of liquid probiotic bacteria solution 2 to be accommodated within it. By way of example, reservoir 4 could have a volume of from 0.5 litres to 200 litres, preferably from 0.6 litres to 2 litres or from 10 litres to 100 litres.

Preferably, the dispensing device 10 comprises stirring means 40 of the liquid probiotic bacteria solution 2 functionally connected to the reservoir 4. The stirring means 40 serve to prevent gradients concentration or deposits from forming within the reservoir.

In the embodiments shown, the stirring means 40 comprise a mechanical stirrer 42 comprising an impeller 44 at least partially immersed - in a rotatable manner - in the liquid solution.

Preferably, the stirring means 40 comprise an insufflation conduit inserted into the liquid solution, configured to introduce a gas (preferably air) into said solution, so as to generate mixing. Preferably, the insufflation conduit has a free end, from which said gas flows out, located at a bottom wall 46 of said reservoir or immersed in said reservoir.

Said reservoir 4 preferably comprises at least one closure element (not illustrated), which overlaps at least partially with an access opening 48 of said reservoir. Said closure element is preferably crossed by openings accommodating the stirring means 40 (i.e. the insufflation conduit or the impeller 44; when provided), a suction conduit 50 of the liquid solution (connected to the peristaltic pump 18) and, optionally, a level detector 52 of said liquid solution.

Reservoir 4, accommodating the liquid solution of probiotic bacteria 2, is preferably a thermostatically controlled reservoir.

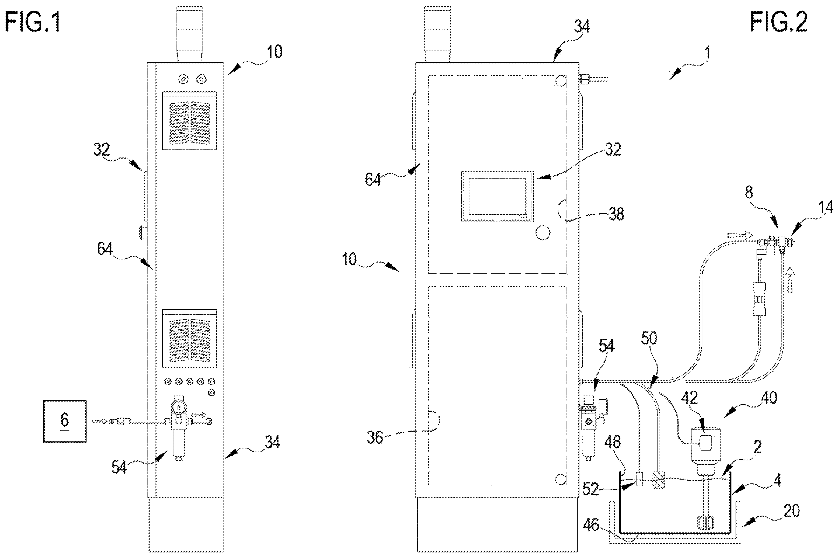

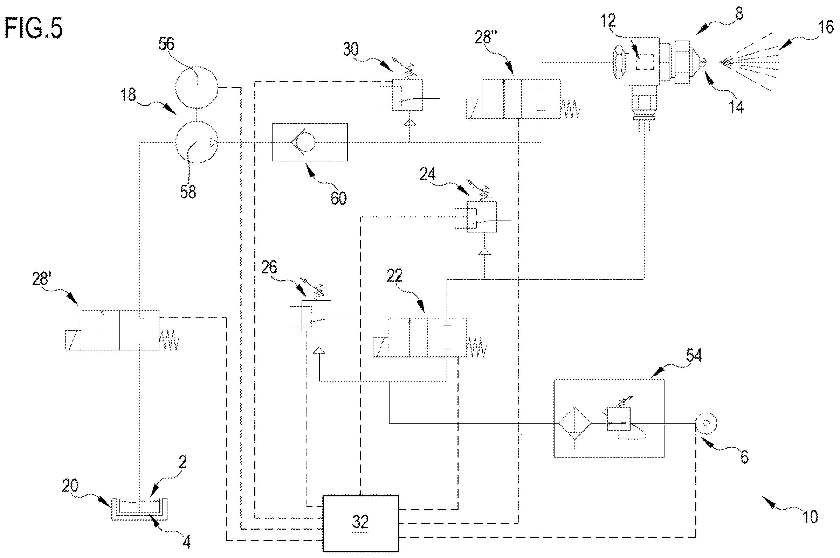

The dispensing device 10 therefore preferably comprises (v) refrigeration means 20 - only schematised in Figures 2 and 5 - in thermal contact with the reservoir 4 to maintain said liquid solution of probiotic bacteria 2 at a predefined temperature. The predefined temperature is preferably from 1°C to 8°C (at a pressure of about 1 atmosphere), more preferably from 2°C to 5°C, even more preferably from 3.5°C to 4.5°C. When the liquid solution of probiotic bacteria 2 is at the predefined temperature, the probiotic bacteria are in a "slowed down" bacterial replication condition (compared to a replication rate at a room temperature of 25°C and about 1 atmosphere).

Preferably, the dispensing device 10 comprises said refrigeration means 20, second liquid solution temperature detection means , and management and control means 32. The management and control means 32 are functionally connected to the second temperature detection means so as to receive temperature detecting signals, and connected to the refrigeration means 20 so as to send temperature control signals to the latter.

Preferably, the means of management and control 32 are electronic. More preferably, the means of management and control 32 comprise at least one programmable logic controller (PLC).

The pressurised gas source 6 is preferably a source of pressurised air (or compressed air).

The source of pressurised gas 6 could include a pressurised container of said gas, or a compressor (fixed or portable), or a compressor plant.

Preferably, the dispensing device 10 comprises a compressed air control unit 54, fluidically placed downstream of the pressurised gas source 6 and upstream of the dispensing body 8. The compressed air control unit 54 serves to regulate a pressurised gas flow and/or to avoid pressure peaks.

The dispensing body 8 delimits a mixing chamber 12 between the liquid probiotic bacteria solution 2 and the pressurised gas, to give a liquid-gas mixture, and comprises at least one dispensing nozzle 14 of said liquid-gas mixture in a form of an atomised jet 16.

Atomised jet 16 comprises substantially spherical droplets with an average diameter in the range from 100 µm to 300 µm.

The average diameter is measured in a manner known in the art, e.g. suitable methods are by means of PDA (Phase-Doppler anemometer) instrumentation.

Preferably, the dispensing nozzle 14 is a nozzle with a variable nozzle opening, i.e. with a passage cross-section that is adjustable depending on *- inter alia* - the pressure and flow rate of the pressurised gas feed and the pressure and flow rate of the liquid solution feed.

As an example, an atomised jet 16 comprising substantially spherical droplets with an average diameter in the range from 100 µm to 300 µm can be obtained under the following conditions:
- liquid solution feed pressure: from 1.8 bar to 2.2 bar, preferably 2 bar;
- pressurised gas feed pressure: from 2.4 bar to 2.6 bar, preferably 2.5 bar;
- liquid solution feed flow rate: from 6.0 I/h to 6.4 I/h, preferably 6.2 I/h;
- pressurised gas feed flow rate: from 49 l/min to 55 l/min, preferably 52 I/min.

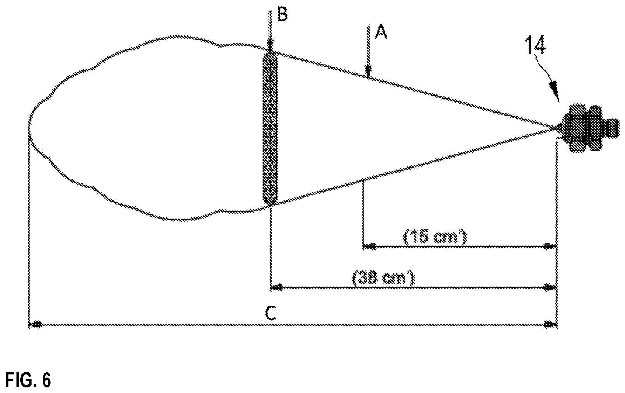

Preferably, the atomised jet 16 has the shape schematised in Figure 6, wherein the parameters A, B, C indicated therein are 38 cm, 53 cm and 3.2 cm, respectively.

The pressure of the pressurised gas exits from the dispensing nozzle is preferably about 2.8 bar and the pressure of the liquid solution exits from the dispensing nozzle is about 2.0 bar.

Preferably, the dispensing nozzle 14 has a conformation characterised by an internal spiral.

Preferably, the dispensing nozzle 14 is removably connected to the dispensing body 8, so that the dispensing nozzle 14 can be replaced according to the passage section required for dispensing the atomised jet 16.

As an example, a dispensing nozzle that can be used in this device or apparatus is an automatic spray nozzle 'SPEEDY JET', model APAEV24VCC, manufactured by EUROSPRAY SPRAY AND FILTER TECHNOLOGY, S.L. (Spain; https://www.euspray.com/).

Peristaltic pump 18 is connected to reservoir 4 to feed the liquid solution of probiotic bacteria 2 to said mixing chamber 12.

The use of a peristaltic pump 18 in the present dispensing device 10 was not a random choice as this type of pump made it possible to mantain the cell membranes of the probiotic bacteria intact, and thus preserve the viability of these bacteria, and also to avoid contamination of the liquid solution before dispensing. Preferably, a compression produced by the peristaltic pump 18 is from 1 atmosphere to 5 atmospheres, preferably from 2 atmospheres to 4 atmospheres.

The peristaltic pump 18 preferably comprises at least one motor unit 56 and at least one rotor unit 58, wherein said rotor is connected to a shaft of the motor unit 56 and comprises a plurality of rollers. By means of a rotation of the rollers, and a translational throttling exerted by said rollers on a flexible portion of the suction conduit 50 (via the peristalsis mechanism), the liquid solution is withdrawn from the reservoir 4 by suction and fed to the mixing chamber 12.

The dispensing device 10 preferably includes, fludically downstream of the peristaltic pump 18, at least one non-return valve 60 of the liquid solution.

The dispensing device 10 preferably comprises: (vi) first intercepting means 22 of the pressurised gas; (vii) first detection means 24, 26 of the pressurised gas feed pressure; (viii) second intercepting means 28', 28" of the liquid probiotic bacteria solution 2; (ix) second detection means 30 of the liquid probiotic bacteria solution 2 feed pressure; and (x) management and control means 32, functionally connected to said (ii), (iv), (vi), (vii), (viii), and (ix).

The management and control means 32 are preferably configured to receive detection signals from (vii) and (ix), and to generate and send control signals to (ii), (iv), (vi) and/or (viii) so that said pressurised gas feed pressure and said liquid probiotic bacteria solution 2 feed pressure are each within predefined ranges.

Preferably, the first detection means of pressurised gas feed pressure 24, 26 comprise a low pressure detector 24 and a high pressure detector 26, the latter being separate and independent from the low pressure detector 24.

Preferably, the second intercepting means 28', 28" of the liquid probiotic bacteria solution 2 comprise a second proximal intercepting means 28', fluidically upstream of the peristaltic pump, and a second distal intercepting means 28", preferably downstream of the peristaltic pump 18 and more preferably upstream of the dispensing body 8.

It is also an object of the present invention a use of said dispensing apparatus 1 for sanitising surfaces and/or environments.

It is also an object of the present invention an air conditioning system comprising said dispensing apparatus 1.

In this system, the atomised jet 16 dispensed by the nozzle 14 is dispensed:
- in one or more ventilation ducts of said air conditioning system; and/or
- at one or more air filters of said air conditioning system; and/or
- together with a flow of thermally treated air and/or displaced from said air conditioning system into a environment or onto a surface.

Said air conditioning system preferably includes or, alternatively, consists of a heating, ventilation and air-conditioning (HVAC) system.

It is also an object of the present invention a method of sanitising. Preferably, said method of sanitising is implemented by said dispensing apparatus 1, or by said air conditioning system.

This sanitisation method comprises the following steps:
(i) dispensing said liquid solution of probiotic bacteria 2 in the form of an atomised jet 16 into an environment or onto a surface;
(ii) sanitising said environment or surface by said probiotic bacteria having intact cell membranes.

Preferably, in step (ii), probiotic bacteria having intact cell membranes antagonistically replicate and colonise said environment or surface.

Advantageously, the apparatus of the present invention makes it possible to maintain the probiotic bacteria in the liquid solution viable during the various stages of storage of the probiotic bacteria and the liquid solution, during the atomisation of said solution, until after the dispensing of said solution in atomised form.

Advantageously, the apparatus object to the present invention makes it possible to minimise the stresses to which probiotic bacteria are subjected, namely: thermal stress, contact stress and compression stress.

In particular, the adoption of a peristaltic pump (preferably electronically controlled) made it possible to reduce or eliminate the consequences of the three above-mentioned stresses, through a safe transit for the bacteria, which circulate from the reservoir to the dispensing nozzle avoiding excessive pressures of the liquid solution, without coming into direct contact with any potentially harmful source of deterioration.

Advantageously, the dispensing nozzle of the dispensing device of the present invention is able to combine two indispensable requirements: on the one hand, to avoid excessive fragmentation of the liquid solution into too small droplets, as this is a source of stress for the probiotic bacteria and their viability. On the other hand, to avoid atomised drops that are too large, as they are unsuitable for suspension transport by airflow.

Advantageously, within the apparatus object of the present invention the probiotic bacteria are able to maintain their characteristics, including their viability, for a period of time of more than 30 days when suspended or resuspended (i.e. reconstituted) in an aqueous solution (preferably of sodium chloride) at a temperature from 1°C to 8°C, preferably from 2°C to 5°C, even more preferably from 3.5°C to 4.5°C. Advantageously, the use of a peristaltic pump in the device object of the present invention does not lead to any kind of alteration in the vital parameters of the probiotic bacteria or the liquid solution comprising them. Advantageously, the dispensing nozzle in the device of the present invention substantially preserves the viability of atomised probiotic bacteria which - downstream of dispensing into the environment or onto the surface remain viable at a percentage from 90% to 100%, preferably from 95% to 99%, even more preferably from 96% to 98.5%.

Advantageously, the dispensing nozzle can dispense a liquid solution of probiotic bacteria in an environment in an amount between 2x10⁵ CFU/m³ and 10x10⁶ CFU/m³ or can dispense a liquid solution of probiotic bacteria on a surface in an amount between 10² CFU/m² and 8x10⁴ /m².

These amounts can be measured by means of the MD8 Airport (Sartorius) air sampler, wherein, starting from a liquid solution of probiotic bacteria in an amount expressed as colony forming units per millilitres (CFU/ml) of liquid solution, it is possible to measure the amount of bacteria dispensed in the form of an atomised jet within a volume expressed in an amount expressed as colony forming units per cubic metre (as m³) of a known volume. Alternatively, using the MD8 Airport (Sartorious) air sampler, starting from a liquid solution of probiotic bacteria in an amount expressed as colony forming units per millilitres (CFU/ml) of liquid solution, it is possible to measure the amount of bacteria dispensed in the form of an atomised jet on a surface expressed in an amount expressed as colony forming units per square metre (as m²) of a known surface.

Advantageously, dispensing apparatus 1 makes it possible to dispense probiotic bacteria in a viable form, in a concentration substantially superimposed on that originally loaded in reservoir 4 of dispensing apparatus 1.

Referring to the above-mentioned embodiments of the apparatus, plant, use and method, a person skilled in the art could make substitutions or modifications to the described characteristics depending on the contingencies. These embodiments are also to be considered within the scope of the invention formalised in the following claims.

Furthermore, it is specified that any embodiment can be implemented independently from the other described embodiments.

### Examples

### Experiment 1. Dispensing of a liquid solution of probiotic bacteria 2 in the form of an atomised jet 16 in an environment

The following experimental methodology was used:
1) Solutions of probiotic bacteria were prepared in phosphate buffered saline (PBS) by dispersing 10⁷ probiotic spores per ml in a volume of 200 ml. Spores in lyophilised form of three different species of bacteria belonging to the genus *Bacillus (B. subtilis, B, pumilus, B. megaterium)* were used.
2) Reservoir 4 of dispensing apparatus 1 was loaded with the prepared solutions, then the instrument was operated until a total volume of 10 ml was collected in a 50 ml sterile tube. The dispensing time required to collect 10 ml was found to be 45 sec.
3) Scalar 1:10 dilutions in PBS (1 ml of suspension in 9 ml of PBS) of both the original solution and the solution collected after the atomising jet was dispensed were then made.
4) Scalar dilutions were inoculated in duplicate on Agar Tryptone Soy (TSA) medium (0.5mL/culture medium)
5) TSA culture media containing scalar dilutions of probiotic bacteria solutions were incubated for 24 h at 37 °C and colony-forming units (CFU) per ml of probiotic bacteria solution were counted.

The results, expressed as CFU/ml of probiotic bacteria solutions, are reported in Table 1, and show that dispensing apparatus 1 is capable of dispensing probiotic bacteria solutions without any loss of viability compared to the original solution (the percentage variations are well within the limits of experimental variability and do not denote any significant loss of concentration and/or viability of the micro-organisms). Dispensing apparatus 1 thus permits the dispensing of probiotic bacteria in a viable form, in a concentration superimposed on that originally loaded into reservoir 4 of dispensing apparatus 1 (maximum variation of 8% with respect to the solution introduced into the reservoir);

**Table 1. Probiotic bacteria dispensing from tested dispensing apparatus 1**

| Species | Initial concentration *(CFU*/*ml)* | Dispensed concentration *(CFU*/*ml)* | *Reduction %* |
|---|---|---|---|
| *B. subtilis* | 7.10 x 10⁷ | 6.90 x 10⁷ | -2 % |
| *B. pumilus* | 5.61 x 10⁷ | 5.12 x 10⁷ | -8 % |
| *B. megaterium* | 2.08 x 10⁷ | 1.90 x 10⁷ | -5% |

### Experiment 2. Dispensing of a liquid solution of probiotic bacteria 2 in the form of an atomised jet 16 in an indoor environment and on a surface in an indoor environment.

Considering the results obtained in the preliminary experiment, the concentration of probiotic bacteria was assessed by dispensing a liquid solution of probiotic bacteria 2 in the form of an atomised jet 16 in a 1 m³ volume chamber (either dispensed in the air or on a surface), at different dispensing times.
1) All liquid solutions of probiotic bacteria used in the test were prepared prior to experimentation as described in Experiment 1
2) Inside a test chamber, 1m x 1m x 1m (1 m³ volume) sized, 4 sterile plastic Petri dishes of 90 mm diameter were placed, fixing them to the bottom of the test chamber and to the walls (at a height of 50 cm), in order to highlight the possible deposition of probiotic bacteria on the plastic Petri dishes surfaces from what was nebulised by dispensing apparatus 1 in the test chamber.
3) Nebulisation of the probiotic suspension inside the test chamber by means of dispensing apparatus 1 then follows, placing the dispenser body 8 directly inside one of the test chamber openings provided for this purpose, and keeping all other test chamber openings hermetically sealed.
4) Dispensing apparatus 1 was operated for 3 different times: 30 seconds, 1 minute and 3 minutes, at the end of which the samples to be analysed were collected; between these times, the chamber was sanitised by peroxidation in order to eliminate the probiotic bacteria already dispensed and return the chamber to its initial decontaminated state.
5) At the times indicated in step 4, the samples were collected as follows: at the end of the three operating times of the control apparatus 1 (30 seconds, 1 minute, 3 minutes), the air inside the chamber was sampled using an MD8 Airport (Sartorius) air sampler, fixing the amount of air at 0.5 m³ (corresponding to 15 minutes of suction) .
6) At the end of air sampling, plates were collected from the surfaces
7) Under a BioHazard Class 2 laminar flow hood, the gelatine collection filters were removed from the air sampler and solubilised in 5 ml of sterile PBS at 37 °C. After completion, 1:10 serial dilutions were performed (1 ml of suspension in 9 ml of PBS).
8) Each scalar dilution was inoculated in duplicate on Agar Tryptone Soy (TSA) culture medium (0.5mL/culture medium).
9) The Agar Tryptone Soy (TSA) culture media inoculated with each scalar dilution were incubated at a temperature of 37 ± 1 °C for 24 h and the colony-forming units (CFU) per of probiotic bacteria solution as m³ of test chamber volume and as m² of sterile plastic Petri dish surface were counted

Table 2 shows the initial concentrations as CFU/ml and the amount of probiotic bacteria in air inside the test chamber as CFU/m³ over a period of operation of dispensing apparatus 1 of 30 seconds, 1 minute, 3 minutes.

Table 3 shows the initial concentrations as CFU/ml and the amount of probiotic bacteria on the surface of sterile plastic Petri dishes inside the test chamber as CFU/m² over a period of operation of dispensing apparatus 1 of 30 seconds, 1 minute, 3 minutes.

The results of Experiment 2 describe, for example, how starting with a liquid solution containing about 10⁷ CFU/ml, dispensing apparatus 1 is able to dispense a concentration of probiotic bacteria of more than about 10⁶ /m³ in the air in 1 minute of dispensing and a concentration of probiotic bacteria of more than 10³ /m² on surfaces in 1 minute of dispensing.

**Table 2. Concentration of probiotic bacteria dispensed at the indicated times: AIR**

| Species | Initial concentration (CFU/ml) | Air (CFU/m³ ) | | |
|---|---|---|---|---|
| | | 30 sec | 1 min | 3 min |
| *B*. *subtilis* | 7.10 x 10⁷ | 3.21 x 10⁵ | 1.98 x 10⁶ | 9.85 x 10⁶ |
| *B. pumilus* | 5.61 x 10⁷ | 2.92 x 10⁵ | 3.08 x 10⁶ | 8.79 x 10⁶ |
| *B. megaterium* | 2.08 x 10⁷ | 2.56 x 10⁵ | 2.02 x 10⁶ | 9.02 x 10⁶ |

**Table 3. Concentration of probiotic bacteria dispensed at the indicated times: SURFACES**

| Species | Initial concentration (CFU/ml) | Surfaces (CFU/m² ) | | |
|---|---|---|---|---|
| | | 30 sec | 1 min | 3 min |
| *B*. *subtilis* | 7.1 x 10⁷ | 113 | 2.561 | 65.687 |
| *B. pumilus* | 5.61 x 10⁷ | 101 | 3.080 | 52.321 |
| *B. megaterium* | 2.08 x 10⁷ | 142 | 3.212 | 73.900 |

### LIST OF REFERENCE NUMBERS

- 1: dispensing apparatus
- 2: liquid solution of probiotic bacteria
- 4: reservoir
- 6: pressurised gas source
- 8: dispensing body or dispensing gun
- 10: dispensing device
- 12: mixing chamber
- 14: dispensing nozzle
- 16: atomised jet
- 18: peristaltic pump
- 20: refrigeration means
- 22: first intercepting means of the pressurised gas
- 24: first detection means of the pressurised gas feed pressure, preferably low pressure detector
- 26: first detection means of the pressurised gas feed pressure, preferably high pressure detector
- 28': second intercepting means of the liquid probiotic bacteria solution, preferably upstream of the peristaltic pump
- 28": second intercepting means of the liquid probiotic bacteria solution, preferably downstream of the peristaltic pump and more preferably upstream of the dispenser body
- 30: second detection means of the liquid probiotic bacteria solution feed pressure
- 32: management and control means
- 34: device casing
- 36: first casing compartment or lower compartment
- 38: second casing compartment or upper compartment
- 40: stirring means
- 42: mechanical stirrer
- 44: impeller
- 46: bottom wall
- 48: access opening
- 50: suction conduit
- 52: level detector, preferably level switch
- 54: compressed air control unit
- 56: motor unit
- 58: rotor units
- 60: non-return valve
- 62: first temperature detection means
- 64: casing door

## Claims

1. A dispensing apparatus (1) comprising:
(a) a liquid solution of probiotic bacteria (2) having intact cell membranes;
(b) a dispensing device (10) comprising:
(i) a reservoir (4) accommodating said liquid solution of probiotic bacteria (2);
(ii) a pressurised gas source (6);
(iii) a dispensing body (8):
- delimiting a mixing chamber (12) between said liquid solution of probiotic bacteria (2) and said pressurised gas, to give a liquid-gas mixture; **characterized in that** said dispensing body (8) comprises at least one dispensing nozzle (14) of said liquid-gas mixture in the form of an atomised jet (16);
(iv) and the dispensing device (10) comprises at least one peristaltic pump (18) connected to said reservoir (4) to feed said liquid solution of probiotic bacteria (2) to said mixing chamber (12).

2. The dispensing apparatus (1) according to the preceding claim, wherein said dispensing nozzle (14) is configured to dispense substantially spherical droplets with an average diameter between 100 µm and 300 µm.

3. The dispensing apparatus (1) according to any of the preceding claims, wherein said dispensing body (8) comprises a dispensing nozzle (14) wherein the passage section is adjustable as a function of pressure and flow rate of said pressurised gas (8) and as a function of pressure and flow rate of said liquid probiotic bacteria solution (2).

4. The dispensing apparatus (1) according to the preceding claim, wherein said probiotic bacteria are non-sporigenic bacteria selected from bacteria of the genus *Lactobacillus, Bifidobacterium, Streptococcus, Lactococcus, Akkermansia, Intestinimonas, Eubacterium, Faecalibacterium, Neisseria, Roseburia, Cutibacterium,* and mixtures thereof.

5. The dispensing apparatus (1) according to any one of the preceding claims, wherein said probiotic bacteria are freeze-dried bacteria and reconstituted in an aqueous liquid solution of probiotic bacteria (2), preferably in an aqueous liquid solution of sodium chloride.

6. The dispensing apparatus (1) according to any one of the preceding claims, wherein said dispensing apparatus (10) comprises:
(v) refrigeration means (20) in thermal contact with said reservoir (4) to maintain said liquid solution of probiotic bacteria (2) at a temperature from 1°C to 8°C, preferably from 2°C to 5°C, even more preferably from 3.5°C to 4.5°C, in a condition of slowed down bacterial replication of said probiotic bacteria.

7. The dispensing apparatus (1) according to any one of the preceding claims, wherein said dispensing apparatus (10) comprises:
(vi) first intercepting means (22) of the pressurised gas ;
(vii) first detection means (24, 26) of the pressurised gas feed pressure;
(viii) second intercepting means (28', 28") of the liquid probiotic bacteria solution (2);
(ix) second detection means (30) of the liquid probiotic bacteria solution (2) feed pressure;
(x) management and control means (32), functionally connected to said (ii), (iv), (vi), (vii), (viii), and (ix), wherein said management and control means (32) are configured to receive detection signals from (vii) and (ix) and to generate and send control signals to (ii), (iv), (vi), and/or (viii) so that said pressurised gas feed pressure and said liquid probiotic bacteria solution (2) feed pressure are each within predefined value ranges;
preferably wherein the management and control means of (32) are electronic, more preferably wherein the management and control means (32) comprise at least one programmable logic controller (PLC).

8. Use of the dispensing apparatus (1) according to any of the preceding claims for sanitising surfaces and/or environments.

9. A air conditioning system comprising the dispensing apparatus (1) according to any one of claims 1-5, wherein said atomised jet (16) dispensed from said dispensing nozzle (14) is dispensed:
- in one or more ventilation ducts of said air-conditioning system; and/or
- at one or more air filters of said air-conditioning system; and/or
- together with a flow of thermally treated air and/or displaced from said air conditioning system into a environment or onto a surface.

10. The air conditioning system according to the preceding claim, **characterised by** comprising or, alternatively, consisting of a heating, ventilation and air conditioning (HVAC) system.

11. A sanitising method implemented by the dispensing apparatus (1) according to any one of claims 1-8, or by the air conditioning system according to claims 9-10;
wherein said method comprises the following steps:
(i) dispensing said liquid solution of probiotic bacteria (2) in the form of an atomised jet (16) into an environment or onto a surface;
(ii) sanitising said environment or surface by said probiotic bacteria having intact cell membranes.

12. A sanitising method implemented by the dispensing apparatus (1) according to claim 11, wherein in step (i) the liquid solution feed pressure is between 1.8 bar and 2.2 bar, preferably is 2 bar, and wherein the pressurised gas feed pressure is between 2.4 bar and 2.6 bar, preferably is 2.5 bar.

13. A sanitising method implemented by the dispensing apparatus (1) according to claim 11 or 12, wherein in step (i) a liquid solution feed flow rate is between 6.0 I/hour and 6.4 I/hour, preferably is 6.2 I/hour, wherein a pressurised gas feed flow rate is between 49 I/minute and 55 I/minute, preferably is 52 I/minute.

14. The method of sanitising according to claim 11, wherein - in step (ii) - probiotic bacteria antagonistically replicate and colonise said environment or said surface.

## Patentansprüche

1. Ein Dispensiergerät (1), umfassend:
(a) eine flüssige Lösung probiotischer Bakterien (2) mit intakten Zellmembranen;
(b) eine Dispensiervorrichtung (10), umfassend:
(i) ein Reservoir (4), das die flüssige Lösung probiotischer Bakterien (2) aufnimmt;
(ii) eine Druckgasquelle (6);
(iii) einen Dispensierkörper (8):
- der eine Mischkammer (12) zwischen der flüssigen Lösung probiotischer Bakterien (2) und dem Druckgas begrenzt, um ein Flüssigkeits-Gas-Gemisch zu ergeben;
**dadurch gekennzeichnet, dass**
der Dispensierkörper (8) mindestens eine Dispensierdüse (14) für das Flüssigkeits-Gas-Gemisch in Form eines zerstäubten Strahls (16) umfasst;
(iv) und die Dispensiervorrichtung (10) mindestens eine Peristaltikpumpe (18) umfasst, die mit dem Reservoir (4) verbunden ist, um die flüssige Lösung probiotischer Bakterien (2) der Mischkammer (12) zuzuführen.

2. Das Dispensiergerät (1) nach dem vorhergehenden Anspruch, wobei die Dispensierdüse (14) konfiguriert ist, um im Wesentlichen sphärische Tröpfchen mit einem durchschnittlichen Durchmesser zwischen 100 µm und 300 µm abzugeben.

3. Das Dispensiergerät (1) nach einem der vorhergehenden Ansprüche, wobei der Dispensierkörper (8) eine Dispensierdüse (14) umfasst, bei der der Durchtrittsquerschnitt als Funktion von Druck und Durchflussrate des Druckgases (8) und als Funktion von Druck und Durchflussrate der flüssigen probiotischen Bakterienlösung (2) einstellbar ist.

4. Das Dispensiergerät (1) nach dem vorhergehenden Anspruch, wobei die probiotischen Bakterien nicht-sporenbildende Bakterien sind, ausgewählt aus Bakterien der Gattung Lactobacillus, Bifidobacterium, Streptococcus, Lactococcus, Akkermansia, Intestinimonas, Eubacterium, Faecalibacterium, Neisseria, Roseburia, Cutibacterium und Mischungen davon.

5. Das Dispensiergerät (1) nach einem der vorhergehenden Ansprüche, wobei die probiotischen Bakterien gefriergetrocknete Bakterien sind und in einer wässrigen flüssigen Lösung probiotischer Bakterien (2), vorzugsweise in einer wässrigen flüssigen Natriumchloridlösung, rekonstituiert sind.

6. Das Dispensiergerät (1) nach einem der vorhergehenden Ansprüche, wobei die Dispensiervorrichtung (10) umfasst:
(v) Kühlmittel (20) in thermischem Kontakt mit dem Reservoir (4), um die flüssige Lösung probiotischer Bakterien (2) auf einer Temperatur von 1 °C bis 8 °C, vorzugsweise von 2 °C bis 5 °C, noch bevorzugter von 3.5 °C bis 4.5 °C, in einem Zustand verlangsamter bakterieller Replikation der probiotischen Bakterien zu halten.

7. Das Dispensiergerät (1) nach einem der vorhergehenden Ansprüche, wobei die Dispensiervorrichtung (10) umfasst:
(vi) erste Absperrmittel (22) des Druckgases;
(vii) erste Erfassungsmittel (24, 26) des Druckgas-Zufuhrdrucks;
(viii) zweite Absperrmittel (28', 28") der flüssigen probiotischen Bakterienlösung (2);
(ix) zweite Erfassungsmittel (30) des Zufuhrdrucks der flüssigen probiotischen Bakterienlösung (2);
(x) Verwaltungs- und Steuerungsmittel (32), die funktional mit den (ii), (iv), (vi), (vii), (viii) und (ix) verbunden sind, wobei die Verwaltungs- und Steuerungsmittel (32) konfiguriert sind, um Erfassungssignale von (vii) und (ix) zu empfangen und Steuersignale zu generieren und an (ii), (iv), (vi) und/oder (viii) zu senden, sodass der Druckgas-Zufuhrdruck und der Zufuhrdruck der flüssigen probiotischen Bakterienlösung (2) jeweils innerhalb vordefinierter Wertebereiche liegen;
vorzugsweise wobei die Verwaltungs- und Steuerungsmittel (32) elektronisch sind, noch bevorzugter wobei die Verwaltungs- und Steuerungsmittel (32) mindestens eine speicherprogrammierbare Steuerung (SPS) umfassen.

8. Verwendung des Dispensiergeräts (1) nach einem der vorhergehenden Ansprüche zur Desinfektion von Oberflächen und/oder Umgebungen.

9. Ein Klimasystem, umfassend das Dispensiergerät (1) nach einem der Ansprüche 1 bis 5, wobei der aus der Dispensierdüse (14) abgegebene zerstäubte Strahl (16) abgegeben wird:
- in einen oder mehrere Lüftungskanäle des Klimasystems; und/oder
- an einem oder mehreren Luftfiltern des Klimasystems; und/oder
- zusammen mit einem Strom thermisch behandelter Luft und/oder verdrängt aus dem Klimasystem in eine Umgebung oder auf eine Oberfläche.

10. Das Klimasystem nach dem vorhergehenden Anspruch, **dadurch gekennzeichnet, dass** es ein Heizungs-, Lüftungs- und Klimasystem (HLK-System) umfasst oder alternativ daraus besteht.

11. Ein Verfahren zur Desinfektion, das durch das Dispensiergerät (1) nach einem der Ansprüche 1 bis 8 oder durch das Klimasystem nach den Ansprüchen 9 bis 10 implementiert wird;
wobei das Verfahren die folgenden Schritte umfasst:
(i) Abgeben der flüssigen Lösung probiotischer Bakterien (2) in Form eines zerstäubten Strahls (16) in eine Umgebung oder auf eine Oberfläche;
(ii) Desinfizieren der Umgebung oder Oberfläche durch die probiotischen Bakterien mit intakten Zellmembranen.

12. Ein Verfahren zur Desinfektion, das durch das Dispensiergerät (1) nach Anspruch 11 implementiert wird, wobei in Schritt (i) der Zufuhrdruck der flüssigen Lösung zwischen 1.8 bar und 2.2 bar liegt, vorzugsweise 2 bar beträgt, und wobei der Druckgas-Zufuhrdruck zwischen 2.4 bar und 2.6 bar liegt, vorzugsweise 2.5 bar beträgt.

13. Ein Verfahren zur Desinfektion, das durch das Dispensiergerät (1) nach Anspruch 11 oder 12 implementiert wird, wobei in Schritt (i) eine Zufuhrdurchflussrate der flüssigen Lösung zwischen 6.0 l/Stunde und 6.4 l/Stunde liegt, vorzugsweise 6.2 l/Stunde beträgt, wobei eine Druckgas-Zufuhrdurchflussrate zwischen 49 l/Minute und 55 l/Minute liegt, vorzugsweise 52 l/Minute beträgt.

14. Das Verfahren zur Desinfektion nach Anspruch 11, wobei - in Schritt (ii) - probiotische Bakterien sich antagonistisch replizieren und die Umgebung oder die Oberfläche besiedeln.

## Revendications

1. Un appareil de distribution (1) comprenant:
(a) une solution liquide de bactéries probiotiques (2) ayant des membranes cellulaires intactes;
(b) un dispositif de distribution (10) comprenant:
(i) un réservoir (4) contenant ladite solution liquide de bactéries probiotiques (2);
(ii) une source de gaz sous pression (6);
(iii) un corps de distribution (8):
- délimitant une chambre de mélange (12) entre ladite solution liquide de bactéries probiotiques (2) et ledit gaz sous pression, pour donner un mélange liquide-gaz;
**caractérisé en ce que**
ledit corps de distribution (8) comprend au moins une buse de distribution (14) dudit mélange liquide-gaz sous la forme d'un jet atomisé (16);
(iv) et le dispositif de distribution (10) comprend au moins une pompe péristaltique (18) reliée audit réservoir (4) pour amener ladite solution liquide de bactéries probiotiques (2) à ladite chambre de mélange (12).

2. L'appareil de distribution (1) selon la revendication précédente, dans lequel ladite buse de distribution (14) est configurée pour distribuer des gouttelettes sensiblement sphériques avec un diamètre moyen compris entre 100 µm et 300 µm.

3. L'appareil de distribution (1) selon l'une quelconque des revendications précédentes, dans lequel ledit corps de distribution (8) comprend une buse de distribution (14) dans laquelle la section de passage est réglable en fonction de la pression et du débit dudit gaz sous pression (8) et en fonction de la pression et du débit de ladite solution liquide de bactéries probiotiques (2).

4. L'appareil de distribution (1) selon la revendication précédente, dans lequel lesdites bactéries probiotiques sont des bactéries non sporogènes choisies parmi les bactéries du genre Lactobacillus, Bifidobacterium, Streptococcus, Lactococcus, Akkermansia, Intestinimonas, Eubacterium, Faecalibacterium, Neisseria, Roseburia, Cutibacterium, et leurs mélanges.

5. L'appareil de distribution (1) selon l'une quelconque des revendications précédentes, dans lequel lesdites bactéries probiotiques sont des bactéries lyophilisées et reconstituées dans une solution liquide aqueuse de bactéries probiotiques (2), de préférence dans une solution liquide aqueuse de chlorure de sodium.

6. L'appareil de distribution (1) selon l'une quelconque des revendications précédentes, dans lequel ledit dispositif de distribution (10) comprend:
(v) des moyens de réfrigération (20) en contact thermique avec ledit réservoir (4) pour maintenir ladite solution liquide de bactéries probiotiques (2) à une température de 1°C à 8°C, de préférence de 2°C à 5°C, encore plus préférentiellement de 3.5°C à 4.5°C, dans un état de réplication bactérienne ralentie desdites bactéries probiotiques.

7. L'appareil de distribution (1) selon l'une quelconque des revendications précédentes, dans lequel ledit dispositif de distribution (10) comprend:
(vi) des premiers moyens d'interception (22) du gaz sous pression;
(vii) des premiers moyens de détection (24, 26) de la pression d'alimentation du gaz sous pression;
(viii) des seconds moyens d'interception (28', 28") de la solution liquide de bactéries probiotiques (2);
(ix) des seconds moyens de détection (30) de la pression d'alimentation de la solution liquide de bactéries probiotiques (2);
(x) des moyens de gestion et de contrôle (32), reliés fonctionnellement auxdits (ii), (iv), (vi), (vii), (viii) et (ix), dans lequel lesdits moyens de gestion et de contrôle (32) sont configurés pour recevoir des signaux de détection provenant de (vii) et (ix) et pour générer et envoyer des signaux de commande à (ii), (iv), (vi) et/ou (viii) de sorte que ladite pression d'alimentation du gaz sous pression et ladite pression d'alimentation de la solution liquide de bactéries probiotiques (2) soient chacune comprises dans des plages de valeurs prédéfinies;
de préférence dans lequel les moyens de gestion et de contrôle de (32) sont électroniques, plus préférentiellement dans lequel les moyens de gestion et de contrôle (32) comprennent au moins un automate programmable industriel (API).

8. Utilisation de l'appareil de distribution (1) selon l'une quelconque des revendications précédentes pour la sanitisation de surfaces et/ou d'environnements.

9. Un système de conditionnement d'air comprenant l'appareil de distribution (1) selon l'une quelconque des revendications 1 à 5, dans lequel ledit jet atomisé (16) distribué par ladite buse de distribution (14) est distribué:
- dans un ou plusieurs conduits de ventilation dudit système de conditionnement d'air; et/ou
- au niveau d'un ou plusieurs filtres à air dudit système de conditionnement d'air; et/ou
- conjointement avec un flux d'air thermiquement traité et/ou déplacé depuis ledit système de conditionnement d'air dans un environnement ou sur une surface.

10. Le système de conditionnement d'air selon la revendication précédente, **caractérisé en ce qu'**il comprend ou, alternativement, consiste en un système de chauffage, de ventilation et de conditionnement d'air (CVC).

11. Un procédé de sanitisation mis en œuvre par l'appareil de distribution (1) selon l'une quelconque des revendications 1 à 8, ou par le système de conditionnement d'air selon les revendications 9 à 10;
dans lequel ledit procédé comprend les étapes suivantes:
(i) la distribution de ladite solution liquide de bactéries probiotiques (2) sous la forme d'un jet atomisé (16) dans un environnement ou sur une surface;
(ii) la sanitisation dudit environnement ou de ladite surface par lesdites bactéries probiotiques ayant des membranes cellulaires intactes.

12. Un procédé de sanitisation mis en œuvre par l'appareil de distribution (1) selon la revendication 11, dans lequel à l'étape (i) la pression d'alimentation de la solution liquide est comprise entre 1.8 bar et 2.2 bar, de préférence est de 2 bar, et dans lequel la pression d'alimentation du gaz sous pression est comprise entre 2.4 bar et 2.6 bar, de préférence est de 2.5 bar.

13. Un procédé de sanitisation mis en œuvre par l'appareil de distribution (1) selon la revendication 11 ou 12, dans lequel à l'étape (i) un débit d'alimentation de la solution liquide est compris entre 6.0 l/heure et 6.4 l/heure, de préférence est de 6.2 l/heure, dans lequel un débit d'alimentation du gaz sous pression est compris entre 49 l/minute et 55 l/minute, de préférence est de 52 l/minute.

14. Le procédé de sanitisation selon la revendication 11, dans lequel - à l'étape (ii) - les bactéries probiotiques se répliquent de manière antagoniste et colonisent ledit environnement ou ladite surface.
